# EUROPEAN PATENT APPLICATION

(11) **EP 3 501 383 A1**
(43) Date of publication of application: **26.06.2019**
(21) Application number: 17210493.7
(22) Date of filing: 22.12.2017
(51) Int. Cl.: A61B 5/00, A61B 5/024, A61B 5/0402, A61B 5/053

(54) **A FLEXIBLE STRAP HAVING ONE OR MORE SENSORS**

(71) Applicant: Nokia Technologies Oy, 02610 Espoo (FI)
(72) Inventor: LASAROV, Harri, 02330 Espoo (FI); ROUVINEN, Jarkko, 02340 Espoo (FI)
(74) Representative: Higgin, Paul

(57) **Abstract**

An apparatus comprising: a flexible strap having an exterior surface; a connection interface; one or more independent interior conductive interconnects, that extend within the flexible strap and are protected by at least the exterior surface of the flexible strap; at least a first movable sleeve covering a first portion of the flexible strap and movable along the flexible strap; and at least a first sensor attached to the first movable sleeve and electrically connected to the connection interface via at least one of the one or more independent interior conductive interconnects.

## Description

### TECHNOLOGICAL FIELD

Embodiments of the present invention relate to a flexible strap having one or more sensors and a method. In particular, they relate to a chest strap for measuring bio-signals.

### BACKGROUND

It has been recognized that the procedure of measuring a subject's bio-signals can change the bio-signals. For example, in a clinical environment, some subjects have a physiological response that changes their bio-signals.

It would be desirable to obtain data concerning a subject's bio-signals over a prolonged time period, during normal activities, outside a clinical environment.

It would be desirable for the subject to become accustomed to or unaware of the measurement system being used.

It would be desirable for the subject to be able to set-up or re-set-up the system for use without the need to return to a clinical environment.

### BRIEF SUMMARY

According to various, but not necessarily all, embodiments of the invention there is provided an apparatus comprising: a flexible strap having an exterior surface; a connection interface; one or more independent interior conductive interconnects, that extend within the flexible strap and are protected by at least the exterior surface of the flexible strap; a first movable sleeve covering a first portion of the flexible strap and movable along the flexible strap; and a first sensor attached to the first movable sleeve and electrically connected to the connection interface via at least one of the one or more independent interior conductive interconnects.

In some but not necessarily all examples, the first sensor is a flexible sensor.

According to various, but not necessarily all, embodiments of the invention there is provided an apparatus comprising: a flexible strap having an exterior surface; a connection interface; one or more independent interior conductive interconnects, that extend within the flexible strap and are protected by at least the exterior surface of the flexible strap; a first flexible, movable, cover means covering a first portion of the flexible strap and movable along the flexible strap to cover other portions of the flexible strap; and a first flexible sensor attached to the first flexible, movable, cover means and electrically connected to the connection interface via at least one of the one or more independent interior conductive interconnects.

In some but not necessarily all examples, the flexible, movable cover means is a first movable sleeve covering a first portion of the flexible strap and movable along the flexible strap.

According to various, but not necessarily all, embodiments of the invention there is provided a method comprising: providing a flexible strap having an exterior surface, a connection interface, one or more independent interior conductive interconnects extending within the flexible strap and protected by at least the exterior surface; providing a first movable sleeve, covering a first portion of the flexible strap and movable along the flexible strap, and comprising a first sensor electrically connectable to the connection interface via at least one of the one or more independent interior conductive interconnects.

According to various, but not necessarily all, embodiments of the invention there is provided examples as claimed in the appended claims.

*The following portion of this 'Brief Summary' section, describes various features that may be features of any of the embodiments described in the foregoing portion of the 'Brief Summary' section. The description of a function should additionally be considered to also disclose any means suitable for performing that function*

In some but not necessarily all examples, the apparatus comprises: multiple independent conductive interconnects; a second movable sleeve covering a second portion of the flexible strap and movable along the flexible strap; and a second sensor attached to the second movable sleeve;
wherein the first sensor is electrically connected to the connection interface via at least one of the multiple independent conductive interconnects; and
wherein the second sensor is electrically connected to the connection interface via at least a different one of the multiple independent interior conductive interconnects.

In some but not necessarily all examples, the flexible strap is configured to fit around a chest of a human subject.

In some but not necessarily all examples, the first sensor is a sensor that is attachable to and detachable from the first movable sleeve and/or the first movable sleeve is a sleeve that is attachable to and detachable from the flexible strap. The first sensor may be replaceable, for example, user-replaceable or subject-replaceable. The sleeve may be replaceable, for example, user-replaceable or subject-replaceable.

In some but not necessarily all examples, the first sensor is a bio-signal sensor.

In some but not necessarily all examples, the first sleeve has connectors on an anterior surface adjacent the flexible strap that are configured to connect with corresponding connectors at different fixed locations on the flexible strap.

In some but not necessarily all examples, the first movable sleeve has one or more contacts on an anterior surface adjacent the flexible strap, each of the one or more contacts being configured to make electrical contact with a respective contact for one of the one or more independent interior conductive interconnects, that extend within the flexible strap. In some but not necessarily all examples, the contact on an anterior surface adjacent the flexible strap and respective contact for one of the one or more independent interior conductive interconnects are urged towards each other.

In some but not necessarily all examples, the apparatus comprises one or more independent conductive interconnects, wherein the first sensor, attached to the first movable sleeve, is electrically connected to the connection interface via a first independent conductive interconnect, wherein the first independent conductive interconnect, where it extends within the flexible strap and is protected by at least the exterior surface of the flexible strap, provides a first independent interior conductive interconnect, and wherein the first independent conductive interconnect has a variable extension. In some but not necessarily all examples, the first independent conductive interconnect has a flexed configuration, such that unflexing increases an extent of the first independent conductive interconnect or the first independent conductive interconnects has a stretchable configuration, such that stretching increases an extent of the first independent conductive interconnect.

In some but not necessarily all examples, the apparatus comprises signal processing circuitry configured to process output signals from the first sensor and control output feedback provided to a user to assist the user in moving the first movable sleeve and first sensor relative to the strap to correctly position the first sensor relative to a subject wearing the apparatus.

In some but not necessarily all examples, the one or more independent interior conductive interconnects, that extend within the flexible strap, are protected by at least the exterior surface of the flexible strap, and are flexible and hidden.

In some but not necessarily all examples, the connection interface is configured to receive a single composite lead.

In some but not necessarily all examples, one or more of the first sensor and the first movable sleeve are flexible.

The use of a flexible strap creates an apparatus that can be comfortably worn for extended periods. Comfort may be increased by using a flexible cover means such as a flexible sleeve. Comfort may be increased by using flexible sensors.

The use of a connection interface and one or more independent interior conductive interconnects creates a simple system that can be easily used by the subject.

The movable cover means or sleeve allows the subject to straightforwardly locate or re-locate the sensor.

Thus measurement data can be obtained over a prolonged time period, during normal activities, outside a clinical environment, using a comfortable and unobtrusive apparatus that can be easily configured by the subject.

### BRIEF DESCRIPTION

For a better understanding of various examples that are useful for understanding the detailed description, reference will now be made by way of example only to the accompanying drawings in which:
Fig 1 illustrates an example of an apparatus from a perspective view;
Figs 2A and 2B illustrate, in cross-sectional view, an example of an apparatus with a sensor at different positions;
Figs 3A and 3B illustrate, in cross-sectional view, an example of an apparatus with a sensor at different positions;
Figs 4A and 4B illustrate examples of an apparatus having multiple sleeves;
Fig 5 illustrates an example of the apparatus in use;
Fig 6A illustrates an example of a replaceable sensor;
Fig 6B illustrates an example of a replaceable sleeve;
Figs 7A, 7B and 7C illustrate different examples in which the distance between the interface and the sensor is increased or decreased by changing the connector/contact point between the sensor and the conductive interconnect;
Figs 8A, 8B and 9A, 9B and 10A, 10B illustrate different examples in which the distance between the interface and the sensor is increased by extending the conductive interconnect;
Fig 11 illustrates an example of the apparatus comprising signal processing circuitry configured to process output signals from the one or more movable sensors;
Fig 12A illustrates an example of a controller;
Fig 12B illustrates an example of a computer program;
Fig 13 illustrates an example of a method.

### DETAILED DESCRIPTION

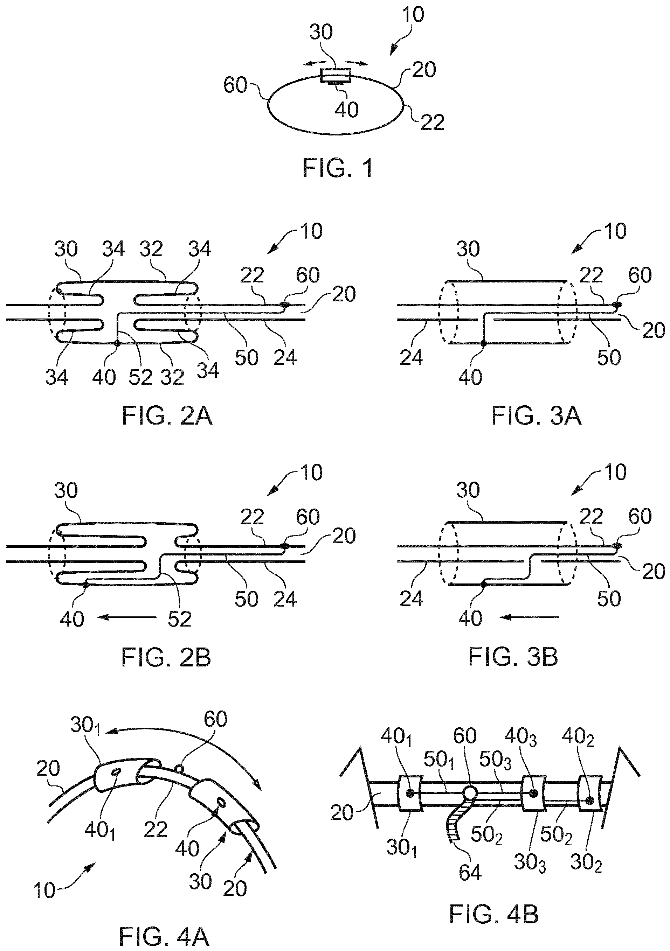

Fig 1, Figs 2A and 2B and Figs 3A and 3B illustrate examples of an apparatus 10 comprising: a flexible strap 20 having an exterior surface 22; a connection interface 60; a first moveable sleeve 30 covering a first portion of the flexible strap 20 and moveable along the flexible strap 20; and a first sensor 40 attached to the first moveable sleeve 30 and electrically connected to the connection interface 60 via at least one independent interior conductive interconnect 50.

The apparatus 10 additionally comprises one or more independent interior conductive interconnects 50, as illustrated in Figs 2A, 2B, 3A, 3B. The one or more independent interior conductive interconnects 50 extend within the flexible strap 20 and are protected by at least the exterior surface 22 of the flexible strap 20.

The first sensor 40 is electrically connected to the connection interface 60 via at least one independent interior conductive interconnect 50.

The moveable sleeve 30 may be moved along the flexible strap 20 to cover different portions of the flexible strap 20. This keeps the conductive interconnect 50 covered.

Although, in this example, a single moveable sleeve 30 is illustrated, in other examples there may be multiple movable sleeves 30. Although, in this example, a single sensor 40 is illustrated, in other examples there may be multiple sensors 40. Although, in this example, a single independent interior conductive interconnect 50 is illustrated in other examples there may be multiple independent interior conductive interconnects 50.

Figs 2A and 2B illustrate one example of an apparatus 10 with the sleeve 30 at different positions and Figs 3A and 3B illustrate a different example of the apparatus 10 with the sleeve 30 at different positions.

Figs 2A, 2B, 3A and 3B are cross-sectional views through the apparatus 10. The cross-section passes through the flexible strap 20, the connection interface 60, the moveable sleeve 30, the first sensor 40 and the one or more independent interior conductive interconnects 50. It will be appreciated that this particular geometry in which these features all lie within the same plane is used for the purposes of illustration. It is of course possible for features not to lie in a common plane.

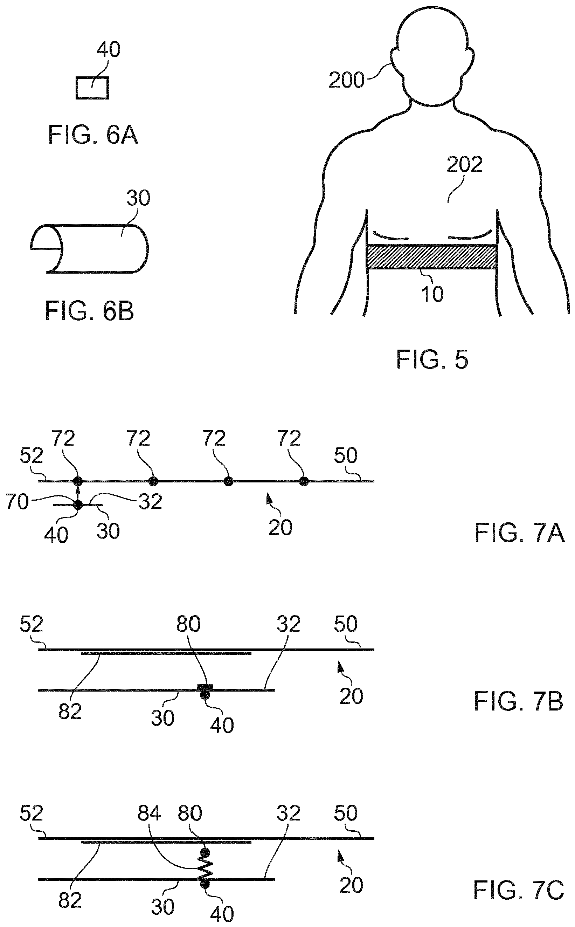

These figures clearly indicate that the one or more independent interior conductive interconnects 50 extend within the flexible strap 20 and are protected by at least the exterior surface 22 of the flexible strap 20. In the examples illustrated, the independent interior conductive interconnects 50 extend between an inner surface 24 of the flexible strap 20 and the exterior surface 22 of the flexible strap 20. The inner surface 24 of the flexible strap 20, in use, is placed adjacent a body of a subject 200 (see Fig 5).

It will be appreciated that in these examples one or more independent conductive interconnects 52 are physically coupled to the connection interface 60 and the first sensor 40. The portion of the one or more independent conductive interconnects 52 that is interior to the strap 10 forms the one or more independent interior conductive interconnects 50. The one or more independent interior conductive interconnects electrically interconnect the first sensor 40 and the connection interface 60.

The interconnects 52, 50 may be formed from any suitable conductor. The interconnect 52 may make a galvanic connection between the connection interface 60 and a sensor 40. The interconnect may, for example, be formed from a wire, laminating, gluing, knitting with conductive metal or textile yarn. The interconnect 52, and interior interconnect 52 may, for example, be elastic, rigid, narrow, wide.
As can be seen from a comparison of Fig 2B to Fig 2A and of Fig 3B to Fig 3A, the sleeve 30 is moved to the left also moving the sensor 40 to the left. This increases the distance between the interface 60 and the sensor 40. However, despite this increase in distance, the sleeve 30 covers the conductive interconnect 52 and where it exits the strap 10. Consequently, the sleeve 30 provides a cover that is moveable to allow the distance between the interface 60 and the sensor 40 to be increased or decreased without the conductive interconnect 52 becoming exposed. Consequently, the one or more independent interior conductive interconnects 50 and the independent conductive interconnects 52 are hidden. In order to maintain the flexibility of the strap 20, it may be desirable for the independent conductive interconnects 52 and the independent interior conductive interconnects 50 to be flexible. In some examples, an electromagnetic shielding may be provided to protect the independent conductive interconnects 52 from external interference or cross interference.

Figs 2A and 2B illustrate an apparatus 10 that is different from that illustrated in Figs 3A and 3B, in that the sleeve 30 is not separate to the strap 20.

In Figs 2A and 2B, the sleeve 30 is formed from the exterior surface 22 of the strap 20. The sleeve 30 comprises a sleeve portion 32 and double-backed interconnecting portions 34 that interconnect the sleeve portion 32 with the exterior surface 22 of the strap 20 that overlies the interior conductive interconnect 50. As can be seen by comparison between Figs 2A and 2B, when the sleeve 30 is moved to the left, the interconnecting portion 32 to the right shortens in length (a shorter double-back) and the interconnecting portion 34 to the left increases in length (a longer double-back). This allows the sleeve portion 32 to be rolled along the strap 20.

In Figs 3A and 3B, the sleeve 30 is separate to the strap 20. The sleeve 30 is not formed from the same exterior surface 22 as the strap 20.

In the example illustrated in Figs 3A and 3B, the sleeve 30 circumscribes a portion of the flexible strap 20. This forms a tube-like enclosure. However, it is not necessary in all examples for the sleeve 30 to entirely enclose the flexible strap 20 (see Fig 6B).

The sleeve may, for example, be formed from textile, plastic, metal or a combination of such materials.

Figs 4A and 4B illustrate examples of an apparatus 10 as previously described. However, in this example the apparatus 10 has multiple sensors 40. In some examples, it is possible to have multiple sensors 40 on a single sleeve 30. However, in this example, multiple sleeves 30 are used so that multiple sensors 40 can be independently positioned.

The apparatus 10 comprises a flexible strap 20 having exterior surface 22; a connection interface 60; multiple independent interior conductive interconnects 50 that extend within the flexible strap 20 and are protected by at least the exterior surface 22 of the flexible strap 20; a first moveable sleeve 30₁ covering a first portion of the flexible strap 20 and moveable along the flexible strap 20 and at least a first sensor 40₁ attached to the first moveable sleeve 30₁ and electrically connected to the connection interface 60 via at least a first one 50₁ of the multiple independent interior conductive interconnects 50. The first independent interior conductive interconnect 50₁ is illustrated in Fig 4B, in which the protective exterior surface 22 of the flexible strap 20 has been removed for purposes of illustration. The protective exterior surface 22, is however illustrated in Fig 4A.

Figs 4A and 4B also illustrate additional moveable sleeves 30. For example, a second moveable sleeve 30₂ covers a second portion of the flexible strap 20 and is moveable along the flexible strap 20. At least a second sensor 40₂ is attached to the second moveable sleeve 30₂ and is electrically connected to the connection interface 60 via at least a second one 50₂ of the multiple independent interior conductive interconnects 50.

A third moveable sleeve 30₃ is illustrated in Fig 4B (but not Fig 4A). The third moveable sleeve 30₃ covers a third portion of the flexible strap 20 and is moveable along the flexible strap 20. At least a third sensor 40₃ attached to the third moveable sleeve 30₃ is electrically connected to the connection interface 60 via at least a third one 50₃ of the independent interior conductive interconnects 50.

Although in this example there is a single sensor for each sleeve 30, it should be appreciated that in other examples there may be multiple sensors 40 and independent interior conductive interconnects 50 for each sleeve 30.

Each of the independent sleeves 30₁, 30₂, 30₃ are independently moveable with respect to the other sleeves. This allows the independent positioning of each sleeve 30 separately and, as a consequence, the independent positioning of a group of the sensors 40 associated with a particular sleeve 30.

Figs 4A and 4B illustrate an example of the shared connection interface 60 that is interconnected to all of the independent interior conductive interconnects 50. The shared connection interface 60 provides a single interface for all of the sensors 40 of the apparatus 10. As illustrated in Fig 4A, the connection interface 60 may be positioned on the exterior surface 22 of the flexible strap 20. As illustrated in Fig 4B, the shared connection interface 60 may be configured to receive a single composite lead 64. The single composite lead 64 may be attached to the shared connection interface 60.

Fig 5 illustrates an example of the apparatus 10 in use. In this example, the apparatus 10 is a portable wearable apparatus that is attached to the subject 200 and moves with the subject 200 who is wearing the apparatus 10. The portable wearable apparatus 10 is connected to the subject 200 and is not physically tethered to any other apparatus not carried by the subject 200. This allows the subject 200 to have freedom of movement.

In this example, the flexible strap 20 is configured to fit around a chest 202 of a human subject 200. The flexible strap 20 is in contact with the chest 202 of the subject 200 at the front, side and back of the chest 202.

The flexible strap 20 is flexible enough so that it can be comfortably worn by the subject 200. It preferably has an adjustable and variable length so that it can be adjusted to fit comfortably around the chest 202 of different subjects 200. This adjustable length may. It is desirable if the portable wearable apparatus 10 is comfortable and can be worn over an extended period of time. It is to be appreciated that the flexible strap 20 may be configured to be worn at any other suitable location of the subject 200, for example around an upper limb (arm or leg), a lower limb (arm or leg) or the neck. It is also to be appreciated that the subject 200 can be an animal. In some examples, more than one of the apparatus 10 may be attached to the subject 200 at one time.

In order to increase comfort, it may be desirable to increase flexibility of the apparatus 10. This may, for example be achieved by using flexible sleeves 30 and/or flexible sensors 40 and/or flexible interconnects 52. Flexible sleeves 30 and/or flexible sensors 40, may, for example, be formed from fabric. For example, it is possible to use conductive fabric to create sensors 40 such as flexible electrodes. Flexible electrodes may also be created using conductive thin films. The electrode material may, for example, comprise metal, plastic, textile, conductive ink or a combination of these.

In some, but not necessarily all examples, it may be desirable for the sleeve 30 to have, on the interior surface 24, a surface that does not easily move relative to the skin of the subject 200 it contacts.

If it is desirable, or necessary to have rigid or semi-rigid portions of the apparatus 10, for example as electronic circuitry, then it may be desirable to include these as portions of the sleeve 30.

In order to fixedly position the sensors 40 relative to the subject 200, it may be desirable to be able to fixedly position the sleeve 30 relative to the strap 20. In some examples, there may be fixtures provided that allows the sleeve 30 to be fixed in a position relative to the strap 20. For example, Velcro™ or a similar non-permanent fixture may be used (see Fig 7A).

Fig 6A illustrates an example of a replaceable sensor 40 that is attachable to and detachable from the moveable sleeve 30. There are connectors on the sleeve 30 that engage with connectors on the sensor 40. The connectors provide not only an electrical connection between the sensor 40 and the respective conductive interconnect 50/52 but also retain the sensor 40 in a fixed position relative to the sleeve 30.

Fig 6B illustrates an example of a moveable sleeve 30 that is a replaceable sleeve 30 that is attachable to and detachable from the flexible strap 20. The replaceable sleeve 30 has connectors on an interior surface of the sleeve 30 for connection to the conductive interconnects 50/52 of the apparatus 10. The replaceable sleeve 30 may have an integrated sensor 40 or it may have a replaceable sensor 40.

In this example, but not necessarily all examples of replaceable sleeves 30, the sleeve 30 does not form a fully enclosing tube that circumscribes entirely the strap 20. In this example, the sleeve 30 forms a partial enclosure that partially circumscribes the strap 20. In other examples of replaceable sleeves 30, the sleeve 30 is configured to form a fully enclosing tube that circumscribes entirely the strap 20.

In the preceding examples, reference has been made to the sensor 40. The sensor 40 may be any type of suitable sensor. It may, for example be an electrode or other type of electrical sensor or it may be a pressure sensor, acoustic sensor, temperature sensor, optical sensor or chemical sensor for example. In some examples a sensor 40 may perform more than one sensing function.

An electrical sensor such as an electrode may be used, for example for electrocardiograph measurements and/or impedance measurements. An optical sensor 40 may for example, be used for photoplethysmography measurements.

Figs 7A, 7B and 7C illustrate different examples in which the distance between the interface 60 and the sensor 40 is increased or decreased without the conductive interconnect 52 becoming exposed. The sleeve 30 covers the conductive interconnect 52. In this example, the distance between the interface 60 and the sensor 40 is increased or decreased by changing the connector/contact point between the sensor 40 and the conductive interconnect 52.

In Fig 7A, the sleeve 30 has connectors 70 on an anterior surface 32 adjacent the flexible strap 20 that are configured to connect with corresponding connectors 72 at different fixed locations on the flexible strap 20. Each of the corresponding connectors 72 forms a physical engagement with the connector 70 on the sleeve 30 and, in addition, forms an electrical interconnection between the sensor 40 on the sleeve 30 and the conductive interconnect 50/52 associated with that sensor. In this example, the sleeve 30 does not necessarily cover all of the connectors 72 which may be positioned on the exterior surface 22 of the strap 20. However, the sleeve 30 does cover the particular connector 72 that is making connection with the connector 70 of the sleeve 30.

The connector 70 and corresponding connector 72 may, for example, be press studs or similar.

In the example of Fig 7B, the first moveable sleeve 30 has one or more contacts 80 on an anterior surface 32 adjacent the flexible strap 20. Each of the one or more contacts 80 is configured to make electrical contact with a respective contact 82 for one or more independent interior conductive interconnects 50 that extend within the flexible strap 20.

As illustrated in this example, the contact 82 and the contact 80 can make electrical contact over an extended area while the sleeve 30 moves relative to the strap 20. In the example illustrated this is achieved by configuring the contact 82 to have a significantly greater area than the contact 80. Of course, the opposite arrangement may also be used in which the contact 80 has a greater area than the contact 82. In this example the electrical contact between the contacts 80 and 82 may be improved by tensioning the flexible strap 20 so that pressure is applied from the strap 20 towards the contact 80 of the sleeve 30. The contact between the contacts 80 and 82 provides an electrical path from the connective interface 60 to the sensor 40 of the sleeve 30. It will be appreciated that in this example the sleeve 30 covers all of the contact 82.

Fig 7C illustrates an example similar to that illustrated in Fig 7B. However, in this example the contact 80 of the sleeve 30 is resiliently biased towards the contact 82 of the strap 20. This resilient bias may, for example, be achieved by using a compressed spring 84. Again, in this example, the sleeve 30 covers the contact 82 during movement of the sleeve 30.

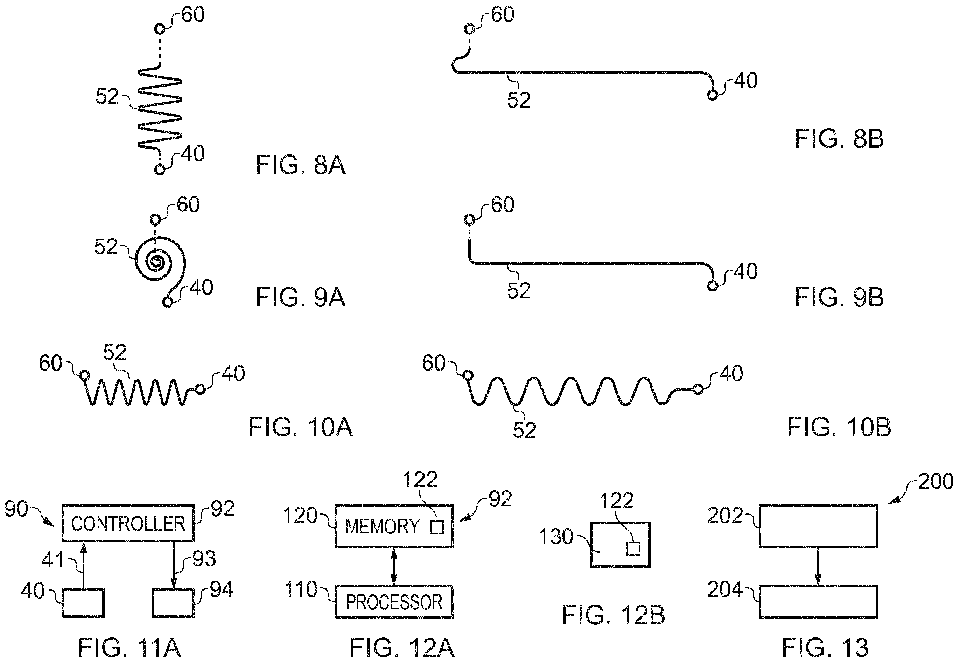

Figs 8A, 8B, 9A, 9B, 10A, 10B illustrate examples in which the distance between the connector interface 60 and the sensor 40 can be increased or decreased without the connective interconnect 52 becoming exposed. In these examples, a sensor 40 attached to a moveable sleeve 30 is electrically connected to the connection interface 60 via an independent conductive interconnect 52. A portion of this independent conductive interconnect or even all of the independent conductive interconnect 52 may be an interior conductive interconnect 50.

The independent conductive interconnect 52 has a variable extension as illustrated in Figs 8A, 8B, 9A, 9B, 10A, 10B. Three examples are illustrated of the conductive interconnect 52 in a non-extended state (Figs 8A, 9A, 10A) and in an extended state (Figs 8B, 9B, 10B). Fig 8B illustrates the conductive interconnect 52 of Fig 8A in an extended state. Fig 9B illustrates the conductive interconnect 52 of Fig 9A in an extended state and Fig 10B illustrates the conductive interconnect 52 of Fig 10A in an extended state.

In Figs 8A and 9A, the independent conductive interconnect 52 has a flexed configuration, such that unflexing increases an extent of the first independent conductive interconnect 52 (Figs 8B, 9B).

In Fig 8A, the conductive interconnect 52 is flexed. It is looped and folded. In some examples the looped/folded portion of the conductive interconnect 52 may be inside the exterior surface 22 of the flexible strap 20 and in other examples it may be located outside the exterior surface 22 of the flexible strap 20 but protected by the sleeve 30.

In Fig 9A, the conductive interconnect 52 is flexed. It is spiraled. In some examples the spiraled portion of the conductive interconnect 52 may be inside the exterior surface 22 of the flexible strap 20 and in other examples it may be located outside the exterior surface 22 of the flexible strap 20 but protected by the sleeve 30.

Figs 10A and 10B illustrate an example in which a conductive interconnect 52 has an un-stretched configuration (Fig 10A) and a stretched configuration (Fig 10B). Stretching increases an extent of the independent conductive interconnect 52.

Fig 11 illustrates an example of the apparatus 10 comprising signal processing circuitry 90 configured to process output signals 41 from the one or more movable sensors 40 and control output feedback 93 provided to the user to assist the user in moving the first moveable sleeve 30 and first sensor 40 relative to the strap 20 to correctly position the first sensor 40 relative to the subject 200 wearing the apparatus 10. In some examples the user and the subject 200 may be the same person.

The signal processing circuitry 90 enables the location of the sensor or sensors 40 to be optimized. In this example the signal processing circuitry 90 is provided by a controller 92, however, in other examples it may be provided by different circuitry.

The signal processing circuitry 90 may be located within the apparatus 10 or it may be connected to the apparatus 10, for example via the connector interface 60. In other examples, the signal processing circuitry 90 may be remote from the apparatus 10.

In some examples the connector interface 60 may be a physical interface that allows a separate apparatus comprising the processing circuity 90 to be connected to the apparatus 10. In other examples, the connector interface 60 may be a wireless interface that allows the apparatus 10 to communicate with the signal processing circuitry 90 without physical connections being made between the signal processing circuitry 90 and the apparatus 10.

The signal processing circuitry 90 may be configured to compare signals 41 received from sensors 40 against previously received signals and/or reference templates to identify when an optimal signal 41 is being received.

Implementation of a controller 92 may be as controller circuitry. The controller 92 may be implemented in hardware alone, have certain aspects in software including firmware alone or can be a combination of hardware and software (including firmware).

As illustrated in Fig 12A the controller 92 may be implemented using instructions that enable hardware functionality, for example, by using executable instructions of a computer program 122 in a general-purpose or special-purpose processor 110 that may be stored on a computer readable storage medium (disk, memory etc) to be executed by such a processor 110.

The processor 110 is configured to read from and write to the memory 120. The processor 110 may also comprise an output interface via which data and/or commands are output by the processor 110 and an input interface via which data and/or commands are input to the processor 110.

The memory 120 stores a computer program 122 comprising computer program instructions (computer program code) that controls the operation of the apparatus 10 when loaded into the processor 110. The computer program instructions, of the computer program 122, provide the logic and routines that enables the apparatus to perform the methods illustrated in Figs13. The processor 110 by reading the memory 120 is able to load and execute the computer program 122.

The apparatus 10 therefore comprises:
at least one processor 110; and
at least one memory 120 including computer program code
the at least one memory 120 and the computer program code configured to, with the at least one processor 110, cause the apparatus 10 at least to perform processing of signals from one or more sensors 40.

As illustrated in Fig 12B, the computer program 122 may arrive at the apparatus 10 via any suitable delivery mechanism 130. The delivery mechanism 130 may be, for example, a non-transitory computer-readable storage medium, a computer program product, a memory device, a record medium such as a compact disc read-only memory (CD-ROM) or digital versatile disc (DVD), an article of manufacture that tangibly embodies the computer program 122. The delivery mechanism may be a signal configured to reliably transfer the computer program 122. The apparatus 10 may propagate or transmit the computer program 122 as a computer data signal.

Although the memory 120 is illustrated as a single component/circuitry it may be implemented as one or more separate components/circuitry some or all of which may be integrated/removable and/or may provide permanent/semi-permanent/ dynamic/cached storage.

Although the processor 110 is illustrated as a single component/circuitry it may be implemented as one or more separate components/circuitry some or all of which may be integrated/removable. The processor 110 may be a single core or multi-core processor.

References to 'computer-readable storage medium', 'computer program product', 'tangibly embodied computer program' etc. or a 'controller', 'computer', 'processor' etc. should be understood to encompass not only computers having different architectures such as single /multi- processor architectures and sequential (Von Neumann)/parallel architectures but also specialized circuits such as field-programmable gate arrays (FPGA), application specific circuits (ASIC), signal processing devices and other processing circuitry. References to computer program, instructions, code etc. should be understood to encompass software for a programmable processor or firmware such as, for example, the programmable content of a hardware device whether instructions for a processor, or configuration settings for a fixed-function device, gate array or programmable logic device etc.

As used in this application, the term 'circuitry' refers to all of the following:
(a) hardware-only circuit implementations (such as implementations in only analog and/or digital circuitry) and
(b) to combinations of circuits and software (and/or firmware), such as (as applicable): (i) to a combination of processor(s) or (ii) to portions of processor(s)/software (including digital signal processor(s)), software, and memory(ies) that work together to cause an apparatus, such as a mobile phone or server, to perform various functions and
(c) to circuits, such as a microprocessor(s) or a portion of a microprocessor(s), that require software or firmware for operation, even if the software or firmware is not physically present.

This definition of 'circuitry' applies to all uses of this term in this application, including in any claims. As a further example, as used in this application, the term "circuitry" would also cover an implementation of merely a processor (or multiple processors) or portion of a processor and its (or their) accompanying software and/or firmware. The term "circuitry" would also cover, for example and if applicable to the particular claim element, a baseband integrated circuit or applications processor integrated circuit for a mobile phone or a similar integrated circuit in a server, a cellular network device, or other network device.

Fig 13 illustrates an example of a method 200 comprising: at block 202 providing a flexible strap 20 having an exterior surface 22, a connection interface 60, one or more independent interior conductive interconnects 50 extending within the flexible strap 20 and protected by at least the exterior surface 22 of the flexible strap 20. And at block 204 providing a first moveable sleeve 30, covering a first portion of the flexible strap 20 and moveable along the flexible strap 20 and comprising a first sensor 40 electrically connectable to the connection interface 60 via at least one of the one or more independent interior conductive interconnects 50.

The blocks illustrated in the Figs 13 may represent steps in a method and/or sections of code in the computer program 122. The illustration of a particular order to the blocks does not necessarily imply that there is a required or preferred order for the blocks and the order and arrangement of the block may be varied. Furthermore, it may be possible for some blocks to be omitted.

Where a structural feature has been described, it may be replaced by means for performing one or more of the functions of the structural feature whether that function or those functions are explicitly or implicitly described.

In some but not necessarily all examples, the apparatus 10 is configured to communicate data from the apparatus 10 with or without local storage of the data in a memory at the apparatus and with or without local processing of the data by circuitry or processors at the apparatus 10.

The data may, for example, be measurement data from the one or more sensors 40 or data produced by the processing of measurement data from the one or more sensors 40.

The data may be stored in processed or unprocessed format remotely at one or more devices. The data may be stored in The Cloud.

The data may be processed remotely at one or more devices. The data may be partially processed locally and partially processed remotely at one or more devices.

The data may be communicated to the remote devices wirelessly via short range radio communications such as Wi-Fi or Bluetooth, for example, or over long range cellular radio links. The apparatus may comprise a communications interface such as, for example, a radio transceiver for communication of data.

The apparatus 100 may be part of the Internet of Things forming part of a larger, distributed network.

The processing of the data, whether local or remote, may be for the purpose of health monitoring, data aggregation, patient monitoring, vital signs monitoring or other purposes.

The processing of the data, whether local or remote, may involve artificial intelligence or machine learning algorithms. The data may, for example, be used as learning input to train an acyclic machine learning network such as a multilayer neural network or may be used as a query input to a machine learning network, which provides a response.

The processing of the data, whether local or remote, may produce an output. The output may be communicated to the apparatus 10 where it may produce an output sensible to the subject such as an audio output, visual output or haptic output.

The recording of data may comprise only temporary recording, or it may comprise permanent recording or it may comprise both temporary recording and permanent recording, Temporary recording implies the recording of data temporarily. This may, for example, occur during sensing or image capture, occur at a dynamic memory, occur at a buffer such as a circular buffer, a register, a cache or similar. Permanent recording implies that the data is in the form of an addressable data structure that is retrievable from an addressable memory space and can therefore be stored and retrieved until deleted or over-written, although long-term storage may or may not occur. The use of the term 'capture' in relation to an image relates to temporary recording of the data of the image. The use of the term 'store' in relation to an image relates to permanent recording of the data of the image.

The term 'comprise' is used in this document with an inclusive not an exclusive meaning. That is any reference to X comprising Y indicates that X may comprise only one Y or may comprise more than one Y. If it is intended to use 'comprise' with an exclusive meaning then it will be made clear in the context by referring to "comprising only one.." or by using "consisting".

In this brief description, reference has been made to various examples. The description of features or functions in relation to an example indicates that those features or functions are present in that example. The use of the term 'example' or 'for example' or 'may' in the text denotes, whether explicitly stated or not, that such features or functions are present in at least the described example, whether described as an example or not, and that they can be, but are not necessarily, present in some of or all other examples. Thus 'example', 'for example' or 'may' refers to a particular instance in a class of examples. A property of the instance can be a property of only that instance or a property of the class or a property of a sub-class of the class that includes some but not all of the instances in the class. It is therefore implicitly disclosed that a feature described with reference to one example but not with reference to another example, can where possible be used in that other example but does not necessarily have to be used in that other example.

Although embodiments of the present invention have been described in the preceding paragraphs with reference to various examples, it should be appreciated that modifications to the examples given can be made without departing from the scope of the invention as claimed.

Features described in the preceding description may be used in combinations other than the combinations explicitly described.

Although functions have been described with reference to certain features, those functions may be performable by other features whether described or not.

Although features have been described with reference to certain embodiments, those features may also be present in other embodiments whether described or not.

Whilst endeavoring in the foregoing specification to draw attention to those features of the invention believed to be of particular importance it should be understood that the Applicant claims protection in respect of any patentable feature or combination of features hereinbefore referred to and/or shown in the drawings whether or not particular emphasis has been placed thereon.

## Claims

1. An apparatus comprising:
a flexible strap having an exterior surface;
a connection interface;
one or more independent interior conductive interconnects, that extend within the flexible strap and are protected by at least the exterior surface of the flexible strap;
a first movable sleeve covering a first portion of the flexible strap and movable along the flexible strap; and
a first sensor attached to the first movable sleeve and electrically connected to the connection interface via at least one of the one or more independent interior conductive interconnects.

2. An apparatus as claimed in claim 1, comprising:
multiple independent conductive interconnects;
a second movable sleeve covering a second portion of the flexible strap and movable along the flexible strap; and
a second sensor attached to the second movable sleeve;
wherein the first sensor is electrically connected to the connection interface via at least one of the multiple independent conductive interconnects; and
wherein the second sensor is electrically connected to the connection interface via at least a different one of the multiple independent interior conductive interconnects.

3. An apparatus as claimed in claim 1 or 2, wherein the flexible strap is configured to fit around a chest of a human subject.

4. An apparatus as claimed in any preceding claim, wherein the first sensor is a sensor that is attachable to and detachable from the first movable sleeve and/or the first movable sleeve is a sleeve that is attachable to and detachable from the flexible strap.

5. An apparatus as claimed in any preceding claim, wherein the first sensor is a bio-signal sensor.

6. An apparatus as claimed in any preceding claim, wherein the first sleeve has connectors on an anterior surface adjacent the flexible strap that are configured to connect with corresponding connectors at different fixed locations on the flexible strap.

7. An apparatus as claimed in any preceding claim, wherein the first movable sleeve has one or more contacts on an anterior surface adjacent the flexible strap, each of the one or more contacts being configured to make electrical contact with a respective contact for one of the one or more independent interior conductive interconnects, that extend within the flexible strap.

8. An apparatus as claimed in claim 7, wherein the contacts on an anterior surface adjacent the flexible strap and respective contact for one of the one or more independent interior conductive interconnects are urged towards each other.

9. An apparatus as claimed in any preceding claim, comprising one or more independent conductive interconnects,
wherein the first sensor, attached to the first movable sleeve, is electrically connected to the connection interface via a first independent conductive interconnect,
wherein
the first independent conductive interconnect, where it extends within the flexible strap and is protected by at least the exterior surface of the flexible strap, provides a first independent interior conductive interconnect,
and wherein the first independent conductive interconnect has a variable extension.

10. An apparatus as claimed in claim 9 wherein the first independent conductive interconnect has a flexed configuration, such that unflexing increases an extent of the first independent conductive interconnect.

11. An apparatus as claimed in claim 9 wherein the first independent conductive interconnect has a stretchable configuration, such that stretching increases an extent of the first independent conductive interconnect.

12. An apparatus as claimed in any preceding claim comprising signal processing circuitry configured to process output signals from the first sensor and control output feedback provided to a user to assist the user in moving the first movable sleeve and first sensor relative to the strap to correctly position the first sensor relative to a subject wearing the apparatus.

13. An apparatus as claimed in any preceding claim, wherein the one or more independent interior conductive interconnects, that extend within the flexible strap, are protected by at least the exterior surface of the flexible strap, and are flexible and hidden.

14. An apparatus as claimed in any preceding claim, wherein the connection interface is configured to receive a single composite lead.

15. An apparatus as claimed in any preceding claim, wherein one or more of the first sensor and the first movable sleeve are flexible.
